# EUROPEAN PATENT APPLICATION

(11) **EP 1 642 584 A1**
(43) Date of publication of application: **05.04.2006**
(21) Application number: 03736248.0
(22) Date of filing: 25.06.2003
(51) Int. Cl.: A61K 31/5575, A61K 45/00, A61K 9/107, A61K 9/14, A61P 15/10, A61P 43/00

(54) **EXTERNAL PREPARATION FOR IMPROVING COITAL FUNCTION**

(71) Applicant: Cardiovascular Institute, Ltd, Ichikawa-shi, Chiba 272-0022 (JP)
(72) Inventor: UCHIDA, Yasumi, FUNABASHI-SHI, Chiba 274-0063 (JP); UCHIDA, Yasuto, FUNABASHI-SHI, Chiba 274-0063 (JP); UCHIDA, Haruko, FUNABASHI-SHI, Chiba 274-0063 (JP)
(74) Representative: Hartz, Nikolai
(86) International application number: PCT/JP2003/008031
(87) International publication number: WO 2005/000316

(57) **Abstract**

The present invention provides a preventive or a remedy for penile erectile dysfunction, which promotes blood-flow in a penile sponge body over long time and thus induces erection while showing high safety without any adverse effect on a body as a whole, when externally administered to a penis.

Use of a drug of the present invention enables to prevent or treat male penile erectile dysfunction and, at the same time, overcome a problem of female insertion impediment caused by increased resistance due to vaginal secretion disorder. Namely, a sexual life of patients (including aged persons) having coital dysfunction can be normalized and thus QOL can be improved.

## Description

### Technical Field

The present invention relates to a topical hydrogel preparation effective for treatment of penile erectile dysfunction and coital dysfunction.

### Background Art

Along with increase in population of the middle and advanced age, the number of patients with erectile dysfunction has increased correspondingly. Also, patients with erectile dysfunction caused by a social factor have increased among the young and those in the prime of their manhood. The major causes of erectile dysfunction include psychological factors, peripheral nerve disorders, endocrine disorders, peripheral circulatory disorders, and side effects of a drug. The rate of patients with erectile dysfunction in Japan is 42% for the age of 55 to 59 years, 65% for 65 to 69 years (Shirai, M., Impotence, 2, 67 ― 93 (1987)), 75% for patients with diabetes (Soh, J., Int. J. Urol., 7 (Suppl. 88), 110 (2000)), and around 68% for patients with hypertension (Burchardt, M., J. Urol., 164, 1188 (2000)) . Also, operative treatment of prostate causes the disease in high rate (Walsh, P.C., J. Urol., 128, 492-497 (1982)).

Penile erection occurs when inner blood pressure of a penile sponge body is increased by higher flow rate of artery blood than that of venous blood. In any one of the above-mentioned causes, erectile dysfunction is brought about by disorder attributable to failure of increasing inner pressure of a penile sponge body. Therefore, medication for increasing artery blood-flow has played a central role in treatment of erectile dysfunction, and so far, injection of a vasodilator drug such as prostaglandin E₁ or papaverine hydrochloride has been applied. However, the administration of such drugs is sometimes dangerous because it may cause systemic blood-pressure drop, bleeding, focal neurological deficit, and the like, and inconvenient because of its short duration of action (Ishii, N., Modern Physician, 19, 1135-1137 (1999)).

Until now, a large number of vasodilator drugs for oral administration have also been attempted, but they have not been effective. Only agents clinically confirmed effective by administration other than injection into a penis were sidenafil and its derivatives which increase blood-flow by promoting generation of nitrogen monoxide (NO) by inhibiting phosphodiesterase 5. However, even sidenafil showed no effect for 30% of patients (Goldenstein, J., N. Engl. J. Med., 338, 1397-1404 (1998)) and side-effects were observed in 6 to 54% cases, and occasional incidences of sudden death from cardiac causes have been reported (McMahon, C.G., J. Urol., 164, 1192-1196 (2000)). In addition, sidenafil has a problem of contraindication to a person who takes a nitrate drug, and therefore, use of sidenafil is limited by itself.

On the other hand, prostaglandin I₂ (hereinafter referred to as PGI₂) derivatives have strong effects on platelet aggregation inhibition and on vasodilation, and are used as a therapeutic agent for peripheral circulatory disorder, and the like. Also, sustained-release preparations comprising a PGI₂ derivative and a base material of a hydrogel have been reported (WO 98/41210, WO 99/33491). However, it has not been known at all that PGI₂ induces erection when administered externally to a penis.

It is an object of the present invention to provide a preventive or a remedy for penile erectile dysfunction which promotes blood-flow in a penile sponge body over long time and thus induces erection while showing high safety without any adverse effect on a body as a whole, when externally administered to a penis. Use of this drug makes it possible to prevent or treat male penile erectile dysfunction, and in consequence, a sexual life of patients (including aged persons) having coital dysfunction can be normalized and thus QOL can be improved.

### Summary of the Invention

Taking into consideration of the above described situation, the present inventors have extensively studied on various medications which may induce penile erection when administered externally to a penis, and found that when a PGI₂ derivative, especially a 4,8-inter-m-phenylene derivative represented by beraprost sodium, is formulated as hydrogel preparation using a hydrogel base material (a gelling agent) such as polysaccharides, proteins or synthetic polymers, absorption of the PGI₂ derivative through the skin or the mucous membrane of a penis is increased dramatically and promotes blood-flow in a penis and thus induces erection. Furthermore, it was found that the relevant hydrogel preparation has good properties of moisture retention and reduction of frictional resistance, and therefore a problem of coital dysfunction due to female vaginal secretion disorder can be solved at the same time.

That is, the present invention provides an external topical hydrogel preparation containing a prostaglandin I₂ derivative for prevention or treatment of penile erectile dysfunction.

Also, the present invention provides an external topical hydrogel preparation containing a prostaglandin I₂ derivative for improving coital dysfunction.

Also, the present invention provides use of a hydrogel which contains a prostaglandin I₂ derivative for a production of an external topical preparation for prevention or treatment of penile erectile dysfunction.

Further, the present invention provides a method for treatment of penile erectile dysfunction, characterized in that a hydrogel which contains a prostaglandin I₂ derivative is administered to a penis or a urethra of a patient.

### Brief Description of the Drawings

Fig. 1 is a schematic representation of an infusion apparatus used for intra-urethral administration of the preparation of the present invention. A: injection syringe type, B: capped cylinder type. 1: a fluted cylinder of an injector (a specific amount of a preparation is pushed out by rotating a cylinder), 2: a preparation containing a cylinder of an injector, 3: a tubing for insertion, 4: a cap, 5: a scale indicating dosage.
Fig. 2 shows a thermograph of temperature change at a glans, a penis and a scrotum before embrocation (left) and at 6 hours after embrocation of the preparation No. 1 (0.5 ml). Left: before embrocation; black arrow: a penis; white arrow: a scrotum; white arrowhead: a glans; +: temperature measurement site (temperature difference before and after embrocation at the measurement site: 4.1°C).
Fig. 3 shows time course of temperature change of a penis after embrocation to a penis with the preparations No. 1, No. 19, No. 21 and No. 61. Numerical character: preparation No.; *: P<0.05; **: P<0.01 vs. preparation No. 61; t: P<0.05 vs. preparation No. 21; PGE₁ is injected into a penis.
Fig. 4 shows a chart of penile oxygen saturation after embrocation with the preparation No. 1. StO₂: oxygen saturation; OXYHb: oxyhemoglobin; DEOXYHb: deoxyhemoglobin; C: before embrocation; 1, 6, 12 and 24h are time after embrocation.
Fig. 5 shows time course of penile oxygen saturation after embrocation to a penis with the preparations of No. 1, No. 21 and No. 61. Numerical character: preparation No.
Fig. 6 shows time course of penile hardness after embrocation to a penis with the preparations of No. 1, No. 21 and No. 61. Numerical character: preparation No.
Fig. 7 shows time course of penile temperature change after intra-urethral administration with the preparations of No. 1, No. 21 and No. 61. Numerical character: preparation No.; *: P<0.05; **: P<0.01 vs. preparation No. 61; t: P<0.05 vs. preparation No. 21.
Fig. 8 shows time course of penile oxygen saturation after intra-urethral administration with the preparations of No. 1, No. 21 and No. 61. Numerical character: preparation No.
Fig. 9 shows time course of penile hardness after intra-urethral administration with the preparations of No. 1, No. 21 and No. 61. Numerical character: preparation No.
Fig. 10 shows presence or absence of early-morning erection after embrocation to a penis with the preparations of No. 1, No. 21 and No. 59.
Fig. 11 shows cross-sectional drawing of a spray apparatus for a powder preparation (left: front view, right: side view). 1: a reservoir cell of the powder preparation; 2: a cell shifter; 3: a blade for opening a sealed thin film of a cell; 4: a push button for gas ejection; 5: a gas container; 6: a spray tubing.

### Detailed Description of the Invention

A preparation of the present invention for preventing or treating penile erectile dysfunction or improving coital dysfunction is a hydrogel preparation comprising a PGI₂ derivative for external administration to a penis.

In the PGI₂ derivative of the present invention, known therapeutic agents for peripheral circulatory disorder, especially, 4,8-inter-m-phenyleneprostaglandin I₂ derivatives (for example, JP-A-1990-12226, JP-A-1990-57548 and JP-A-1989-53672) are preferably included, and more specifically, for example, beraprost, limaprost, iloprost, clinprost, ataprost, ciprostene, naxaprostene, taprostene, cicaprost, pimilprost, CH-169 and SM-10902, or the salts thereof are included. Among them, from a view point of penile blood-flow promotion and induction of penile erection, beraprost sodium ( (+)-(1R*,2R*,3aS*,8bS*)-2,3,3a,8b-tetrahydro-2-hyd roxy-1-[(E)-(3S*)-3-hydroxy-4-methyl-1-octene-6-ynyl] -1H-cyclopenta [b] benzofuran-5-butanoic acid sodium salt) is particularly preferable.

The PGI₂ derivative of the present invention may be synthesized according to a known method (JP-A-1983-124778, JP-A-1994-62594), or may be obtained by extraction and purification from commercially available medicinal products.

Content of the PGI₂ derivative in the hydrogel preparation of the present invention is preferably about 0.00001 to 1% by weight of total weight of the preparation, and 0.0001 to 0.01% by weight is particularly preferable.

When the PGI₂ derivative is externally administered alone or as an oil-based ointment preparation, effects of penile blood promotion and induction of erection are slight or not observed, however, when formulated as a hydrogel preparation, the PGI₂ derivative presents excellent effects of penile blood promotion and induction of erection. Therefore, the preparation of the present invention is useful as an external preparation for preventing or treating penile erectile dysfunction.

Furthermore, the relevant hydrogel preparation exhibits, at the same time, outstanding effects of moisture retention and reduction of frictional resistance. If an embrocation site dries out in a short time after topical application of the preparation, the drug may exfoliate. Therefore, embrocation just before getting into sexual intercourse is essential. Also, if the female side has a problem with decreased vaginal secretion, insertion of a penis may sometimes become difficult. Therefore, the hydrogel preparation of the present invention having effects of moisture retention and reduction of frictional resistance in addition to the activity of inducing erection of a penis may be a coital improving drug for smooth sexual intercourse.

In the hydrogel preparation of the present invention, a substance to be used for forming hydrogel (a gelling agent) is a high-molecular-weight compound which can form a hydrogel particle by absorbing water and swell when contacted with water, and includes, for example, polysaccharides, proteins, synthetic polymers, specifically seaweed extracts or uronic acids as their components such as agar, carrageenan, furcelleran, alginate, kelp extract and fucoidan; plant seeds mucilages such as guar gum, locust bean gum, polysaccharides of tamarind seed, tara gum and cassia gum; plant fruit mucilages such as pectin and arabinogalactan; bacterial mucilages such as xantan gum, pullulan, dextran and gellan gum; mucopolysaccharides such as chondroitin sulfate, hyaluronic acid or its salt and dermatan sulphate; animal proteins such as gelatin, albumin, casein and atelocollagen; plant proteins such as soybean proteins and wheat proteins; cellulose or its derivatives such as carboxymethylcellulose, methylcellulose and microcrystalline cellulose; starch and its derivatives such as starch, starch phosphate and starch glycolate; synthetic polymers such as polyvinyl alcohol, polyacrylate and polyethylene glycol, and it is preferable that one or more kinds thereof are used in combination.

In particular, from the point of activities of penile blood-flow enhancement, penile erectile induction and frictional resistance reduction, use of seaweed extracts such as alginate, kelp extract and fucoidan; mucopolysaccharides such as chondroitin sulfate and hyaluronate; animal proteins such as gelatin and atelocollagen; cellulose derivatives such as carboxymethylcellulose and methylcellulose is preferable, and use of kelp extract, fucoidan, hyaluronate (e.g. sodium salt) alginate (e.g. sodium salt) and carboxymethylcellulose is particularly preferable, and use of combination of two or more kinds thereof is further preferable.

As to quantity of a gelling agent to be combined in the hydrogel preparation of the present invention, in consideration of the activities of inducing penile erection, moisture retention, reduction of frictional resistance, and the like, about 0.1 to 50% by weight of the total weight of the preparation is preferable, and about 5 to 20% by weight is more preferable.

In the hydrogel preparation of the present invention, a buffering agent belonging to, for example, a family of lactate, phosphate, citrate or tartarate may be formulated to increase stability of the composition. The hydrogel preparation of the present invention is desirably adjusted to pH 6.0 to 8.0, preferably pH 7.4, using these buffering agents, as appropriate.

In addition, the hydrogel preparation of the present invention may be formulated with, for example, a moisturizing agent, an auxiliary agent for solubilization, a stabilizing agent, a flavoring agent and a colorizing agent, and further, if necessary, for the purpose of enhancing transdermal absorption or reducing frictional resistance, or enhancing comfortable use feeling, for example, sugar alcohols, polyhydric alcohols such as erythritol, xylitol, sorbitol, glycerine, diglycerine, butylene glycol, propylene glycol and polyethylene glycol; inorganic salts such as potassium chloride and sodium chloride; and hydrophilic petrolatum may be formulated. Especially when the formulation is a liniment, formulating with a hydrophilic petrolatum is preferable.

Also, in the hydrogel preparation of the present invention, medicinal components having capability of promoting blood-flow and enhancing sexual function, for example hormone preparations, aphrodisiac drugs, spermatogenic promoters, nitric monoxide-producing agents, and phosphodiesterase 5 inhibitors may be formulated within the range of safety. The relevant components includes, for example, testosterone propionate (androgenic hormone preparation), yohimbine hydrochloride (aphrodisiac drug), amino acids (lysine: spermatogenic promoter, arginine: nitric monoxide-producing agent, and the like), and Sidenafil (phosphodiesterase 5 inhibitor).

The hydrogel preparation of the present invention, in addition to the gel preparation made by dissolving and gelatinizing the above components in water, may also be a powdered gel preparation composed of fine particles of each component with 5 to 30 µm (micrometer) in diameter. In case of powdered gel preparation, direct embrocation of the preparation to a topical site may results in gelatinization by bodily secretion at the relevant site or by water added as appropriate. To produce the powdered gel preparation, when needed, known lubricants (e.g. talc, stearic acid), binders (e.g. starch, dextrin), diluents (e.g. starch, lactose, glucose, sucrose); coloring materials, preservatives, and the like may be added.

Production of the hydrogel preparation of the present invention may be performed according to a method for producing gel preparation known to those skilled in the art, which is to say, for example, the PGI₂ derivative is dissolved in water, then a gelling agent is dispersed in the solution, other components are added, if needed, and dissolved, and then the solution obtained is left at room temperature and gelatinized.

Also, to produce a powdered gel preparation, for example, the PGI₂ derivative, a gelling agent and a diluting agent, if needed, may be mixed and homogenized to make fine particles.

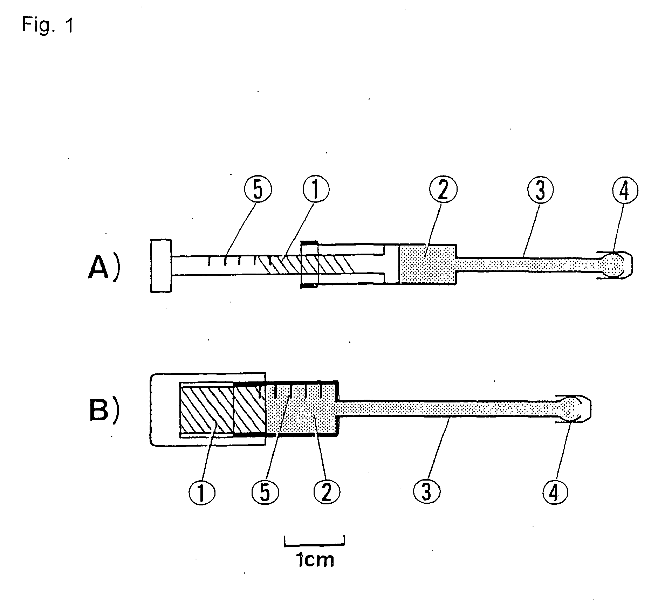

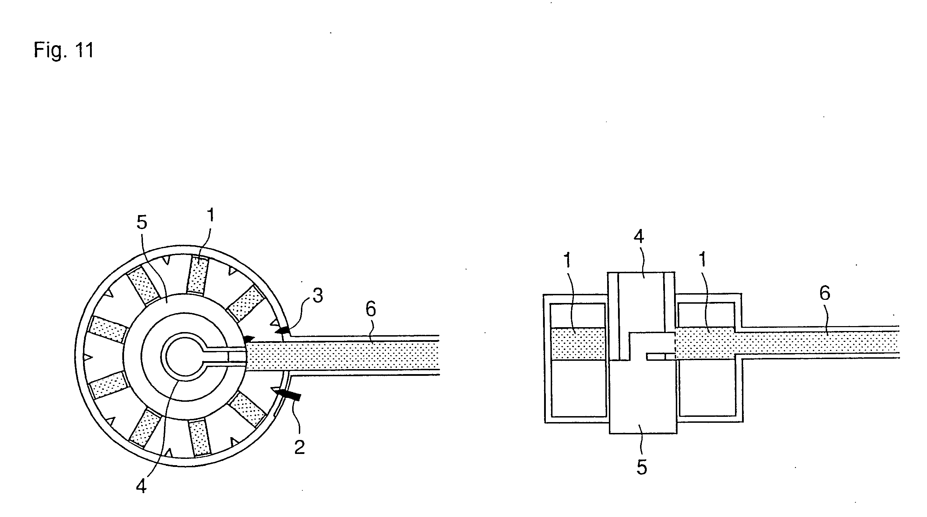

The hydrogel preparation of the present invention is an external preparation to be applied mainly to a penile area including a glans, a foreskin, a neck region and a penis stem, a scrotum and a urethra, and may take optional form suitable for applying to a dermis or to a mucosal membrane of an affected site. In addition, in case of intra-urethral administration, infusion of the preparation may be carried out, when needed, directly or using an apparatus which can be inserted into a urethra such as a flexible tubing (refer to Fig. 1), an extrusion bottle, and the like. In case of the powdered gel preparation, embrocation of the preparation to a topical site can be performed effectively using a spray apparatus for the powder preparation (refer to Fig. 11).

The application dose of the hydrogel preparation of the present invention is, in case of administration by embrocation, 0.1 to 5 g per dose, preferably 0.5 to 1.0 g per dose; in case of administration by intra-urethral infusion, 0.1 to 5.0 g per dose, preferably 0.25 to 0.5 g per dose.

### Examples

Some tests exemplifying the present invention are described below, however, the scope of the present invention should not be limited thereto.

### Example 1

### Production of a hydrogel preparation

Under ambient temperature, a mixture of 100 µg of beraprost sodium, 1 ml of seaweed extract and 0.5 g of sodium hyaluronate was added with a physiological saline solution to make total volume of 10 ml, then mixed homogeneously by stirring for about 30 minutes and gelatinized to produce the preparation No.1.

In addition, as to preparation of seaweed extract, 10 g of raw seaweed root was added to 5 ml of distilled water and mushed using a food mixer for 5 minutes to obtain 15 ml of a solution, followed by centrifugation at 5000 rpm for 30 minutes. The resulted transparent sticky liquid (4 ml) was used as seaweed extract.

Beraprost sodium was prepared by extraction from commercially available tablet pharmaceutical ("Dorner", Toray-Yamanouchi).

By the same way as described above, the preparations (of the present invention) listed in Tables 1 to 5 were prepared.

Each component in the Table was a commercially available product.

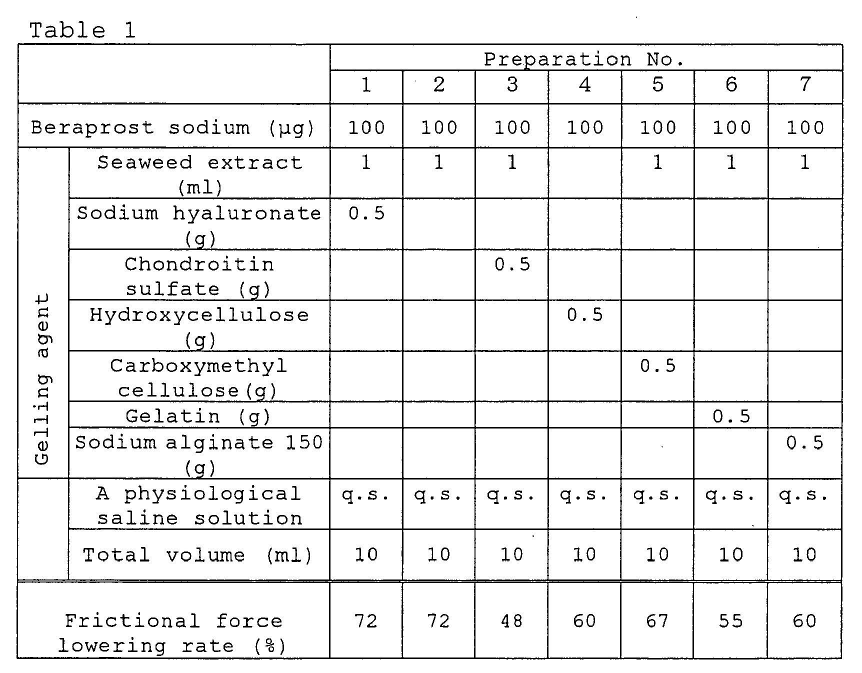

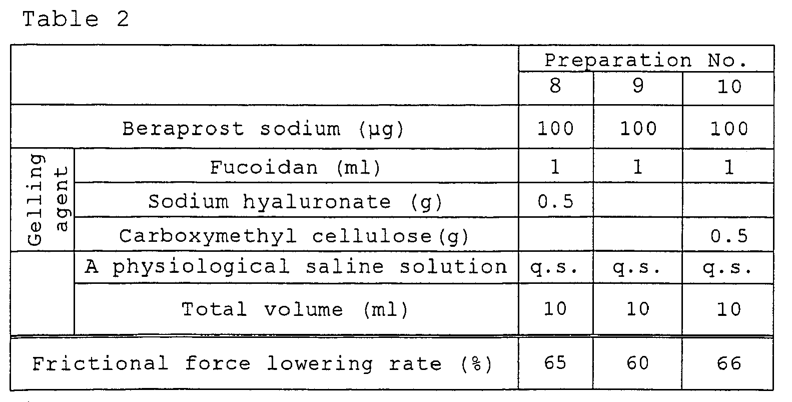

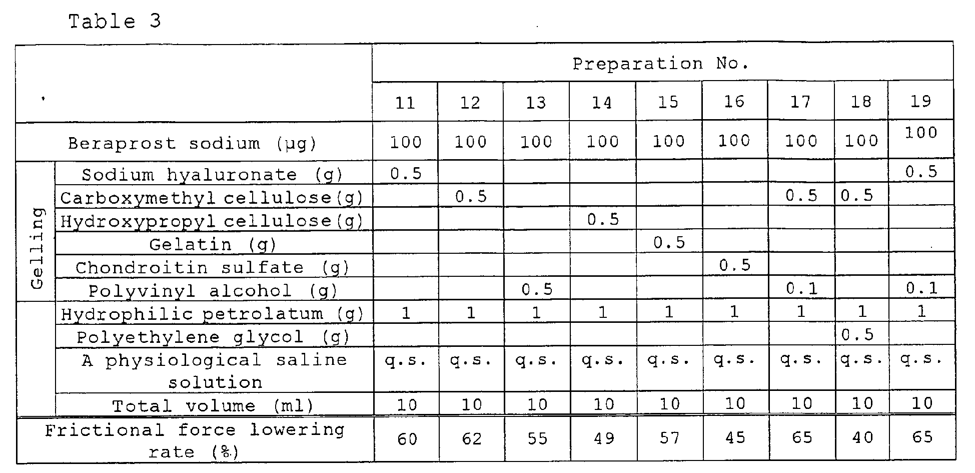

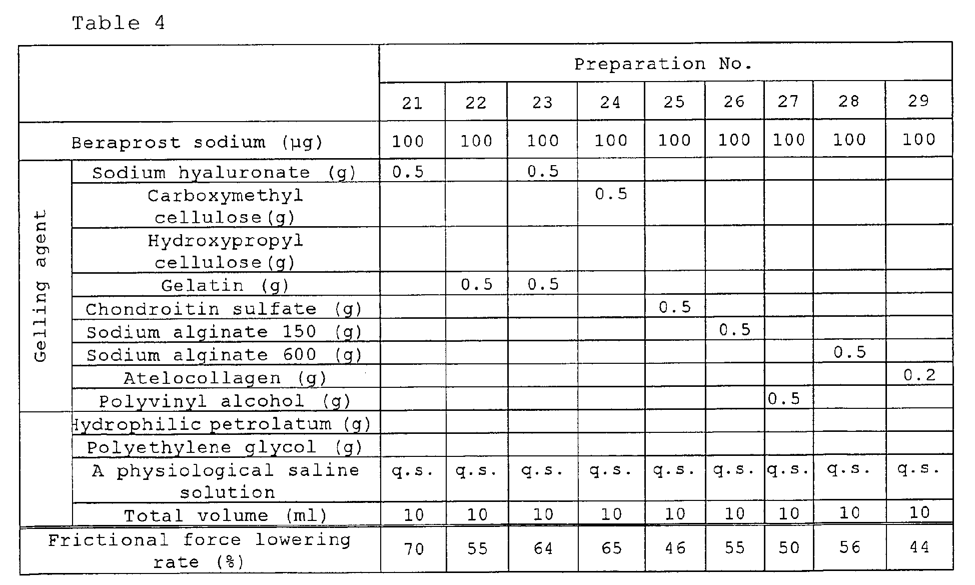

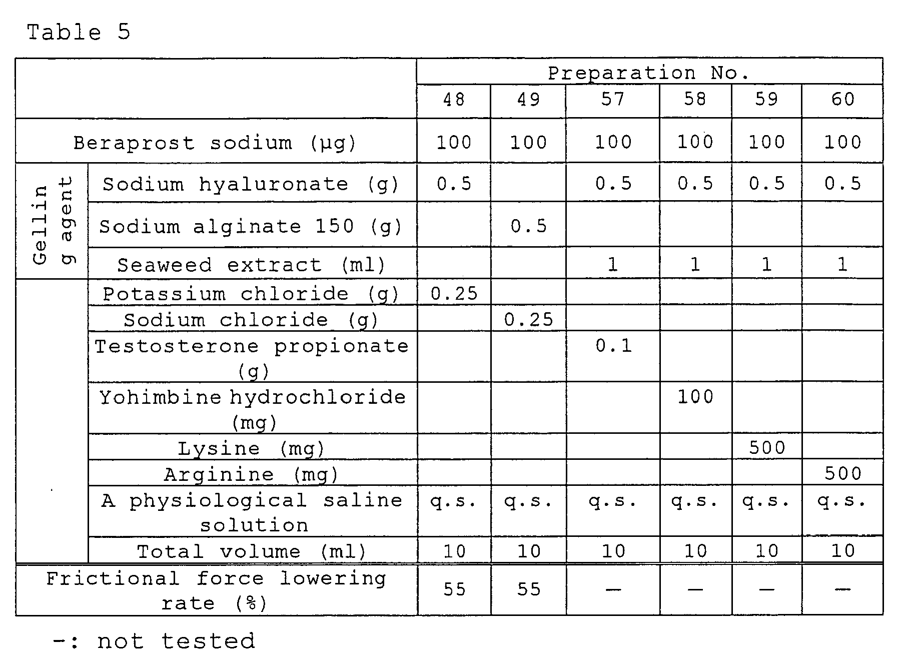

In addition, preparations listed in Tables 6 to 11 (comparative preparations) were prepared as comparison.

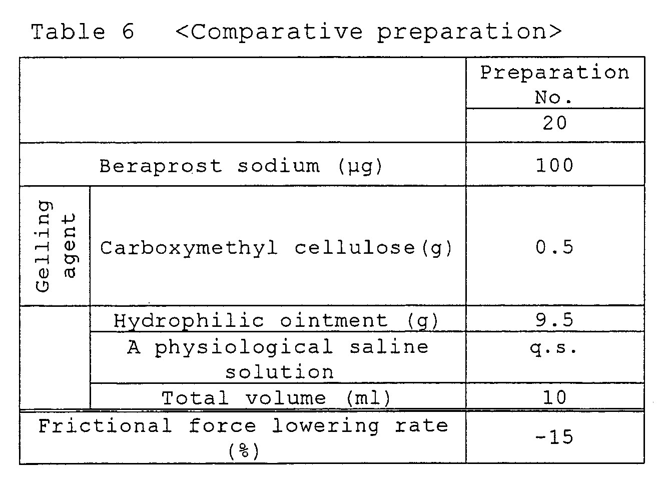

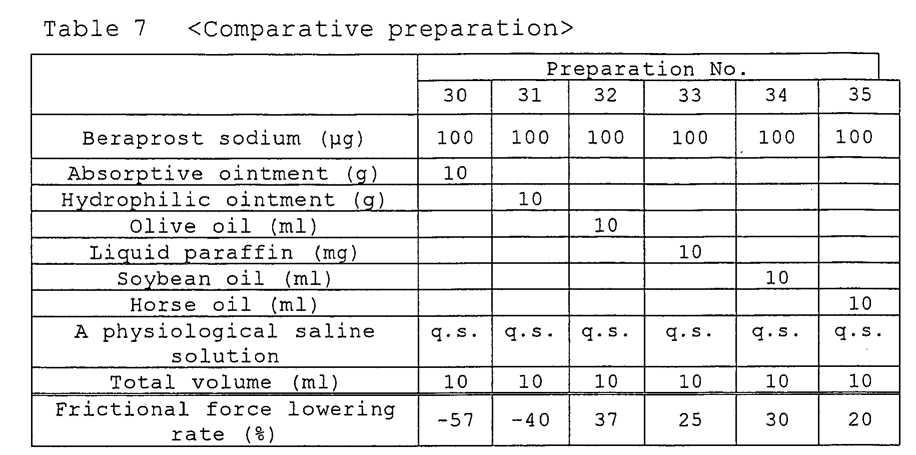

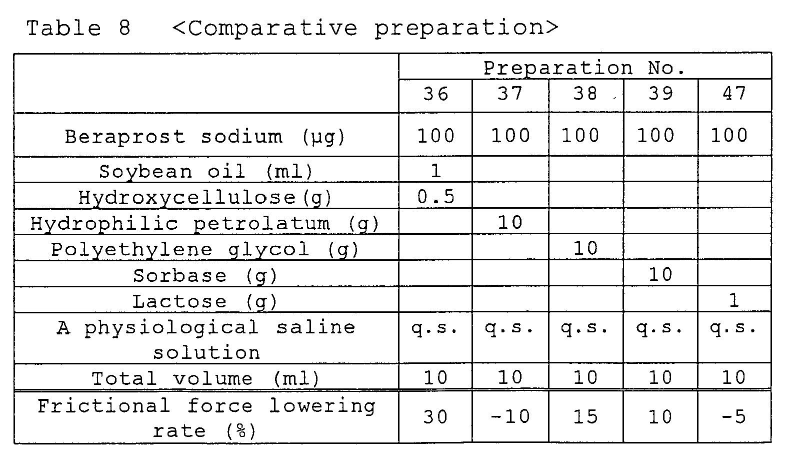

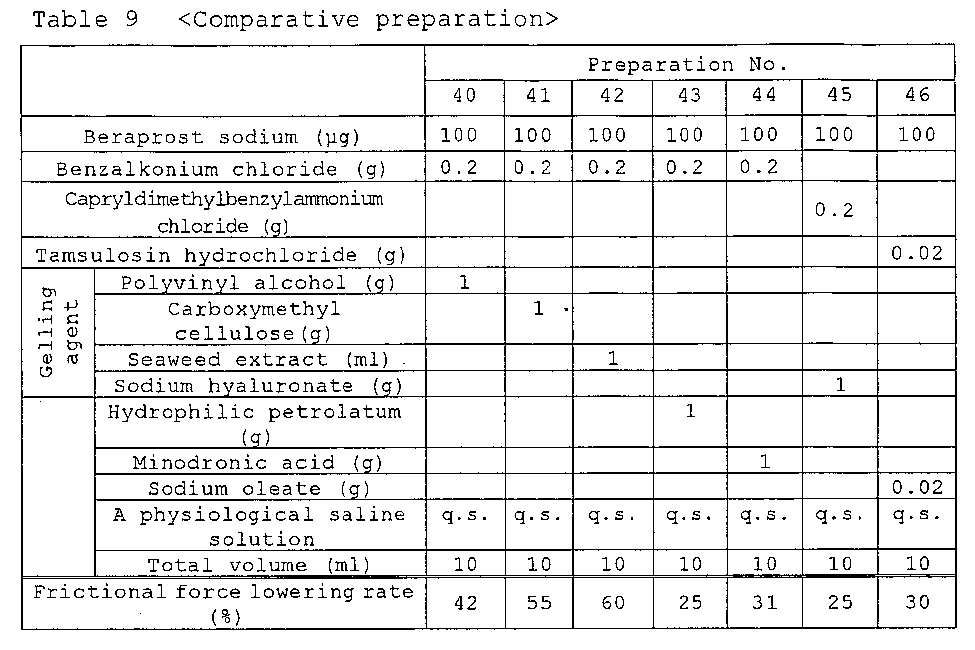

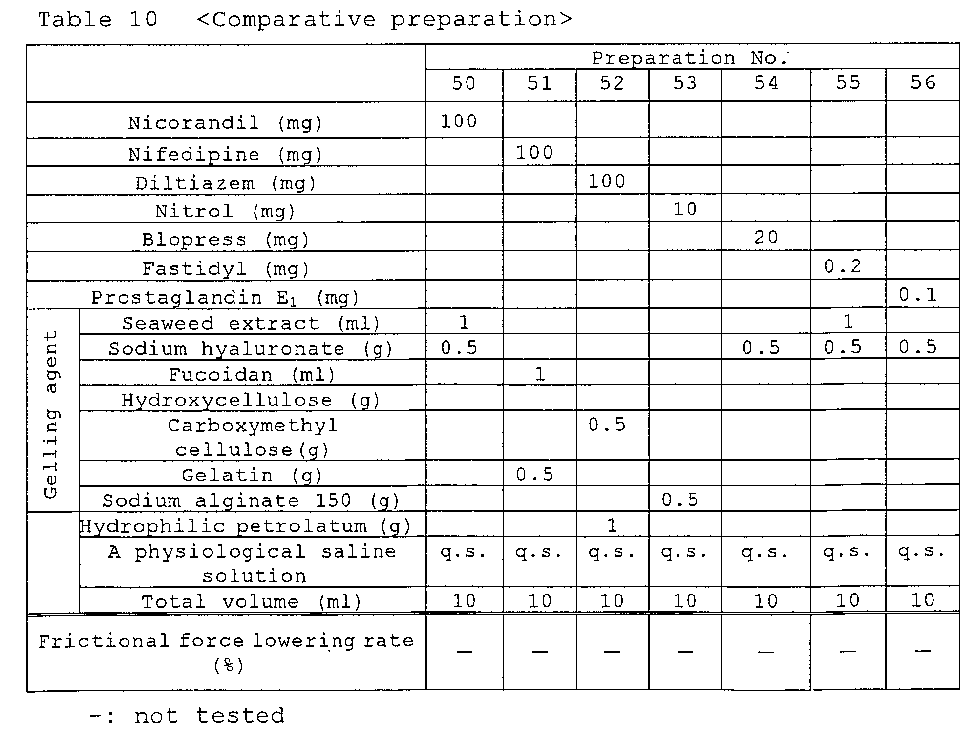

**Table 11 <Comparative preparation>**

| | Preparation No. | |
|---|---|---|
| | 61 | 62 |
| Beraprost sodium (µg) | 100 | |
| Limaprost (µg) | | 100 |
| A physiological saline solution | q.s. | q.s. |
| Total volume (ml) | 10 | 10 |
| Frictional force lowering rate (%) | 8 | 5 |

### Example 2

### Studies on the reduction effect of frictional resistance

To improve a problem of increased vaginal friction due to decreased vaginal secretion, it is preferable that the above-mentioned preparations have effect to reduce frictional resistance. So, in vitro studies were carried out.

Method: An aliquot of 0.5 ml of sample preparations were placed on a glass deck of a microscope, wherein the water soluble or water miscible preparations were diluted 10 times with a physiological saline solution and mixed well, while the water immiscible preparations were as it was. A 5 g balance weight with smooth bottom surface was placed on the sample and linked with a tension meter (Nihon Kohden Corporation) by tying with a piece of thread. The thread was pulled slowly using micromotion equipment and tension generated was recorded on a recorder (NEC San-ei Instruments, Ltd.). Frictional resistance was defined as the maximal tension arisen just before the balance weight started to move. The lowering rate of frictional resistance was calculated by comparison with that obtained using a physiological saline solution as a reference. That is, higher lowering rate means higher friction-lowering effect. The results are also shown in Tables 1 to 11.

Significant frictional resistance-lowering rate was observed in the preparations containing seaweed extract, fucoidan, sodium hyaluronate or carboxymethyl cellulose as a gelling agent, and also observed in the preparations added with hydrophilic petrolatum.

### Example 3

### Clinical studies

(1) Subjects
Five (5) healthcare workers of 60 to 69 years old having no early-morning erection were subjected to the study. After obtaining verbal informed consent, the study was carried out from May 2001 through February 2003 as described below.
After keeping the subjects at rest with naked from the waist down for 5 minutes at room temperature of 25°C and humidity of 60%, temperature of a penis, oxygen saturation in a penile sponge body, and hardness of a penis were measured. The measurement was carried out before administration of the preparation as reference and at 1, 3, 6, 12, 24 and 36 hours after administration, and the value of change (changed value ± average error) were recorded. When there was no change at 3 hours after administration, the preparation was considered not effective and measurement thereafter was quitted.
(2) Administration method
Embrocation: 0.5 ml of each preparation was applied all over to a glans and a penis.
Intra-urethral infusion: An infusion apparatus having a soft silicon tubing on the tip was devised as shown in Fig. 1. The relevant infusion apparatus was inserted into a urethra, and 0.5 ml of the preparation was infused by rotating a cylinder.
(3) Measurement method
1) Measurement of penile temperature change
   Temperature rise reflects increased blood-flow. So, penile temperature was measured by a thermography (Thermo Tracer TH5108, NEC San-ei Instruments, Ltd.). As this instrument can display a measurement site and temperature of the site numerically on a screen, measurements before and after administrations were possible to be performed at the same position.
2) Measurement of blood oxygen saturation (StO₂) in a penile sponge body
   When artery blood-flow in a penile sponge body is promoted, oxygen saturation will increase. So, oxygen saturation in a penile sponge body was measured using a near-infrared spectroscopy. That is, the concentrations of oxyhemoglobin (OXYHb) and deoxyhemoglobin (DEOXYHb) at the position of 3 to 10 mm in depth were measured, and then based on it, a search unit of the near-infrared spectrometer (model PSA-III, Biomedical Science) which can automatically detect oxygen saturation was attached to the left middle dorsum of a penis and measurement was started. The values obtained before and after administration of the preparation were compared.
3) Measurement of penile hardness
   When a penis becomes erected, its hardness increases correspondingly. So, a search unit of a hardness meter (Hardnesstester SD6-11, Mitutoyo Corporation) was pressed against the left dorsum of a penis, and hardness was measured and indicated by mm to compare before and after administration of the preparation.
   In addition, 20 µg of prostaglandin E₁ was injected into a penile sponge body, and hardness was measured by the same way. The value obtained was compared as well.

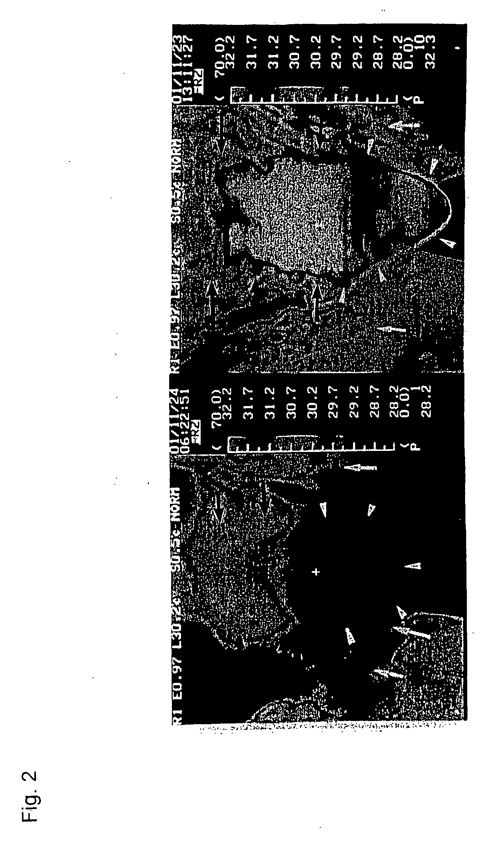

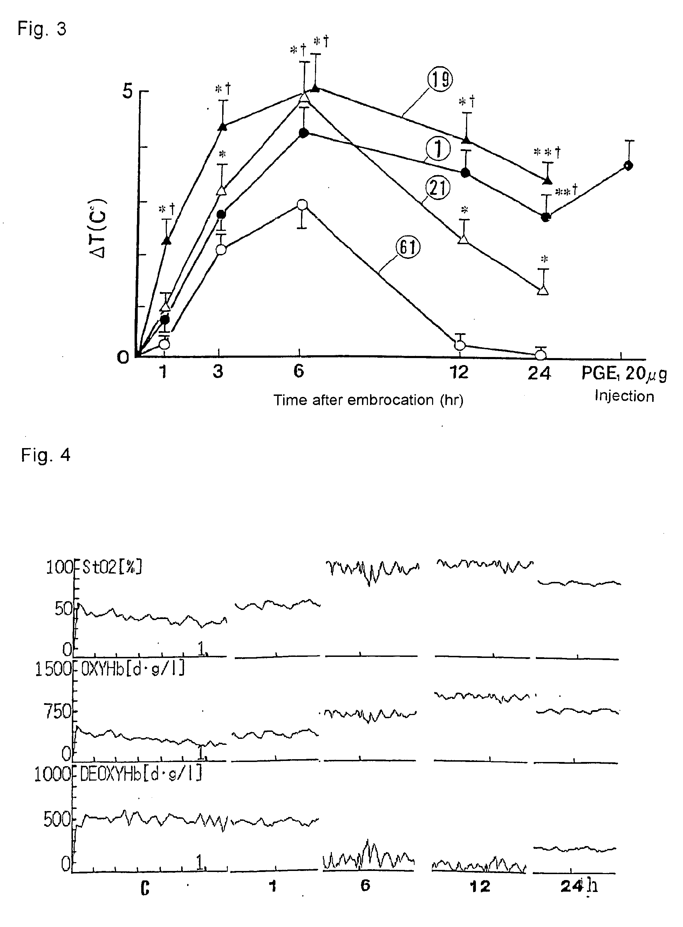

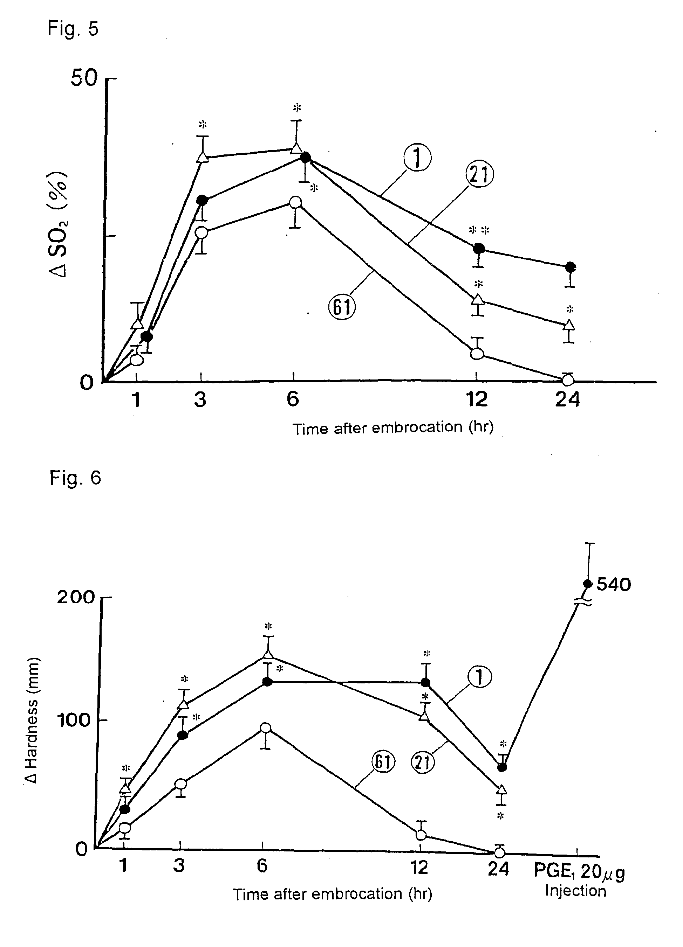

(4) Results
(A) Effect of penile embrocation
(a) Penile temperature change (Figs. 2, 3, Table 12)
The real examples of penile temperature measured by a thermography were shown in Fig. 2. The color appeared on the thermograph indicates: black when temperature is low, while the color changes to purple, red, yellow, pink and white with increase in temperature. Figure in the left-hand shows temperature before embrocation. Temperatures of a glans and a penis are lower than those of an abdomen and a femoral region. At 6 hours after embrocation of the preparation No. 1, as shown in the right-hand, temperatures of a glans and a penis were elevated prominently. Temperature of a scrotum was also elevated.
Time course of 5 cases of penile temperature change after embrocation of main preparations was shown in Fig. 3. Temperature increase by the preparation No. 61 containing beraprost sodium alone ended in 12 hours, however, the preparation No. 1 formulated with seaweed extract, sodium hyaluronate and a physiological saline solution, the preparation No. 21 formulated with sodium hyaluronate and a physiological saline solution and the preparation No. 48, which comprises additional potassium chloride showed that the maximal temperature reached was significantly high compared with the preparation of beraprost sodium alone, and further the duration of action was prolonged for more than 24 hours. In addition, the preparation No. 48 added with potassium chloride showed earlier start of temperature increase. Namely, absorption promoting effect was observed.
(b) Change in oxygen saturation in a penis (Figs. 4, 5, Table 12)
The real examples of time course of oxygen saturation in a penis after embrocation with the preparation No.1 are shown in Fig. 4. In this example, increase in oxygen saturation was retained for more than 24 hours. In Fig. 5, time courses of oxygen saturation in a penis after embrocation with the representative preparations were shown.
(c) Penile hardness change (Fig. 6, Table 12)
Time courses of penile hardness change after embrocation of the representative preparations were shown in Fig. 6.
The preparation No. 1 and No. 21 had effect in maintaining increased hardness for more than 24 hours. However, the increase level was lower compared with that of injection of prostaglandin E₁ into a penis. 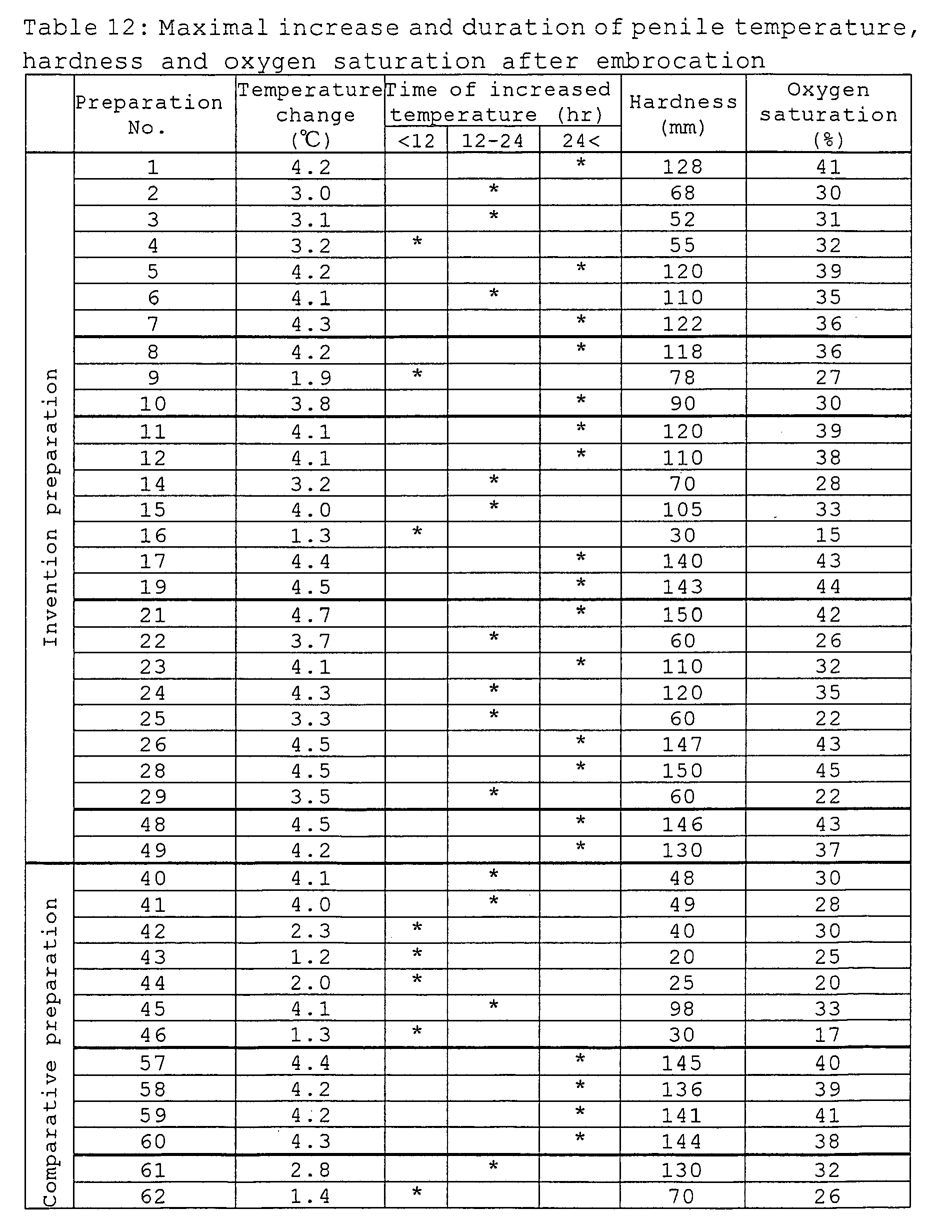
4) The effect of embrocation on a forearm skin
Whether a promotion of blood-flow by embrocation is specific to a penis or not was studied. Namely, the preparations listed in Table 13 below were embrocated on the thin skin of the flexor surface of a forearm, and temperature change was measured. As the result, the preparations which showed prominent temperature increase by embrocation on a penis did not show increase in temperature of a forearm skin (Table 13).

**Table 13: The temperature change by embrocation of the combination drug to a forearm skin**

| Preparation No. | Maximal temperature change of a skin (°C) |
|---|---|
| 1 | 0.2 |
| 5 | 0.3 |
| 7 | -0.1 |
| 8 | 0.3 |
| 10 | 0.2 |
| 11 | 0.1 |
| 12 | -0.2 |
| 21 | 0.3 |
| 26 | 0.1 |
| 61 | 0.4 |

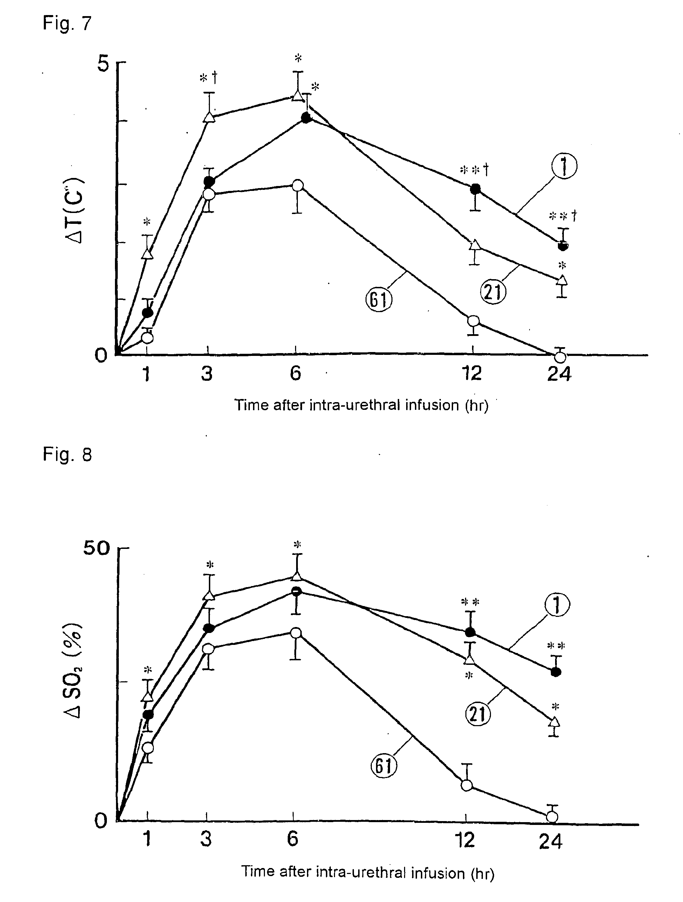

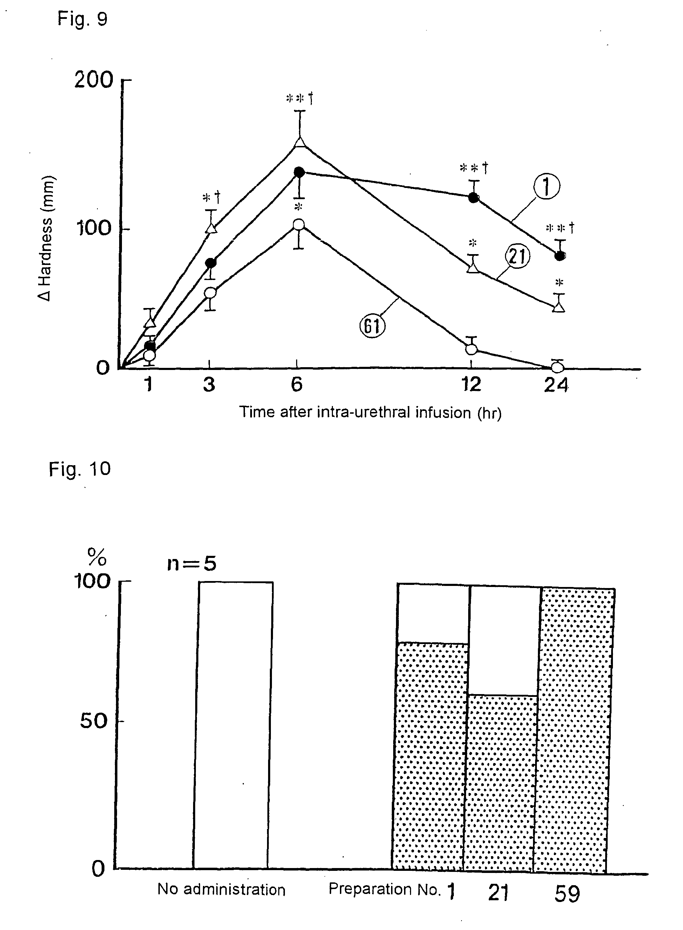

(B) Effect of intra-urethral infusion
(a) Penile temperature change (Fig. 7, Table 14)
   Time courses of penile temperature change after intra-urethral infusion of the representative preparations were shown in Fig. 7.
   Level and duration of temperature increase were low by beraprost sodium alone; however, the preparation No. 12 and No. 26 raised the level of temperature increase and significantly prolonged the duration time.
(b) Change in oxygen saturation in a penis was shown in Fig. 8 and Table 14, and a penile hardness change was shown in Fig. 9 and Table 14.

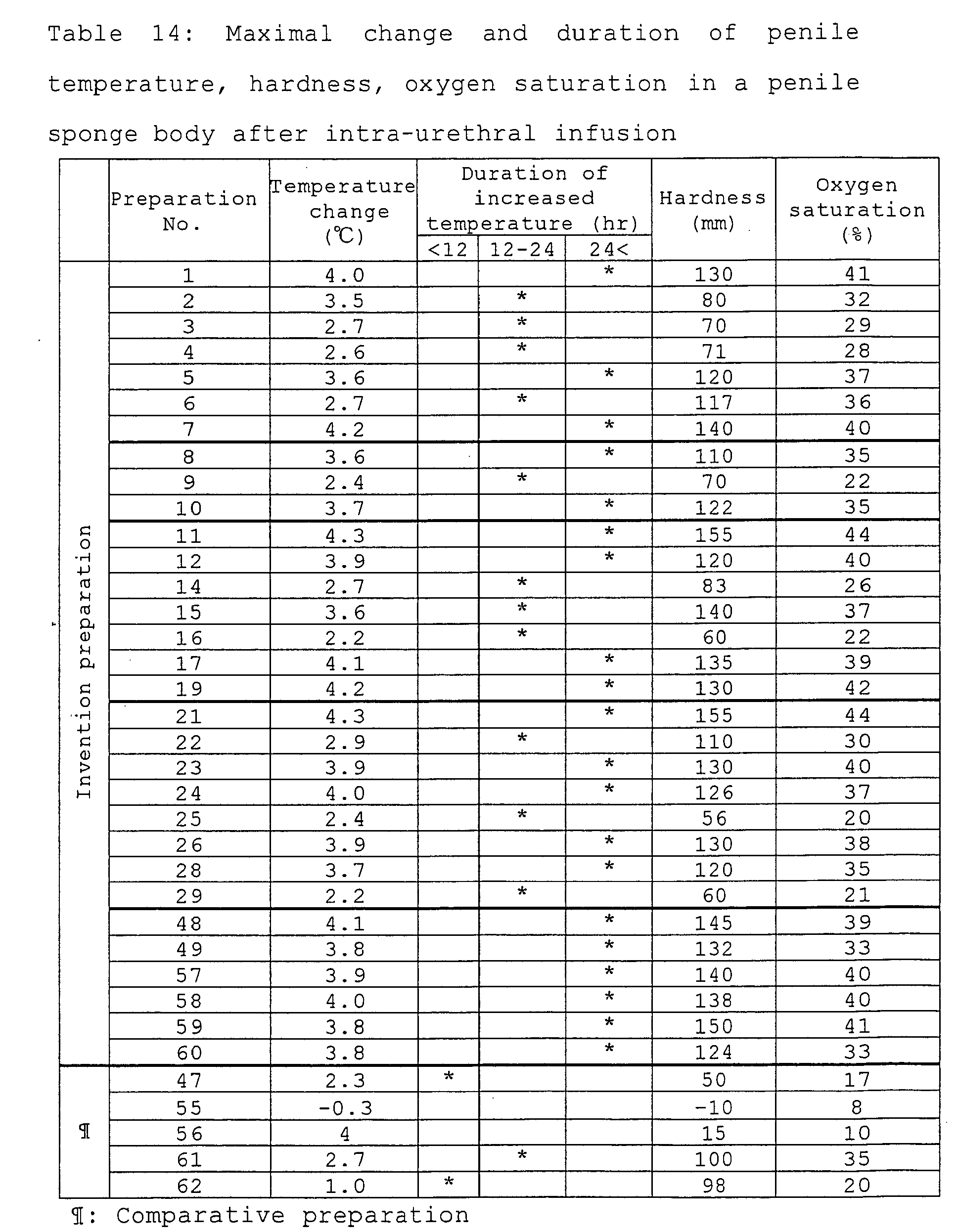

5) Study on early-morning erection
   Early-morning erection is a good indicator of ability of erectile function. And so, 5 cases having no early-morning erection were tested whether or not they would get it back by continuous embrocation of the preparations for 3 days. If the subject could have early-morning erection at least once, the preparation was qualified as effective.
   The result is shown in Fig. 10. The preparation No. 1 and No. 21 induced erection, and the preparation No. 59 which was added with lysine induced erection in all cases.
6) Studies on exfoliation and adverse reaction of the preparations
   In case of external preparation, if the embrocated preparation is exfoliated from a local site by drying out, effects of blood-flow promotion and friction reduction will be declined. Further, if the preparation has some adverse effects, it can not be used for clinical treatment. And so, studies on the exfoliation, adverse reaction (pain, erosion, bleeding) and contradiction were carried out. The results are shown in Tables 15 and 16 together with the summary of blood-flow promotion and induction of penile erection. 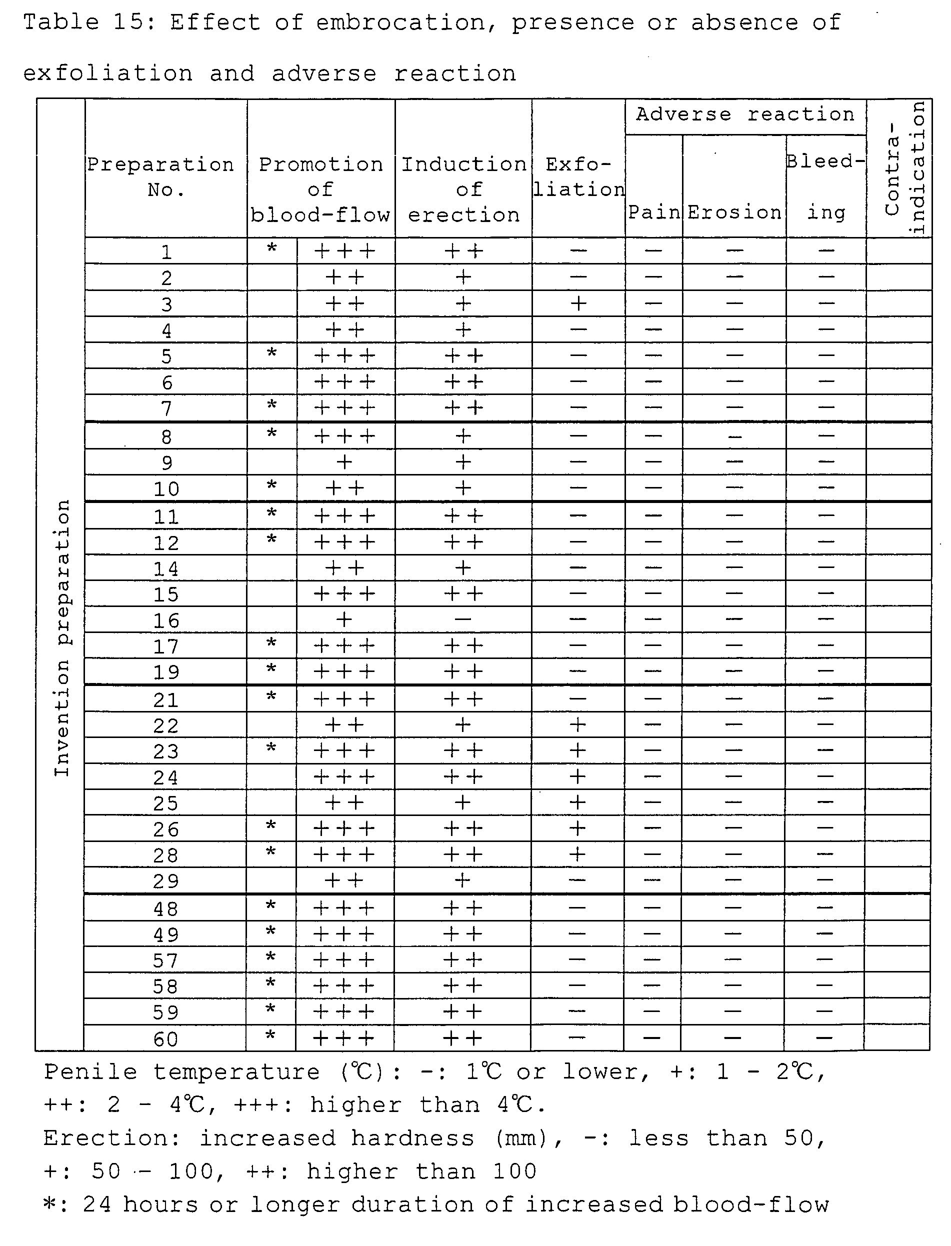 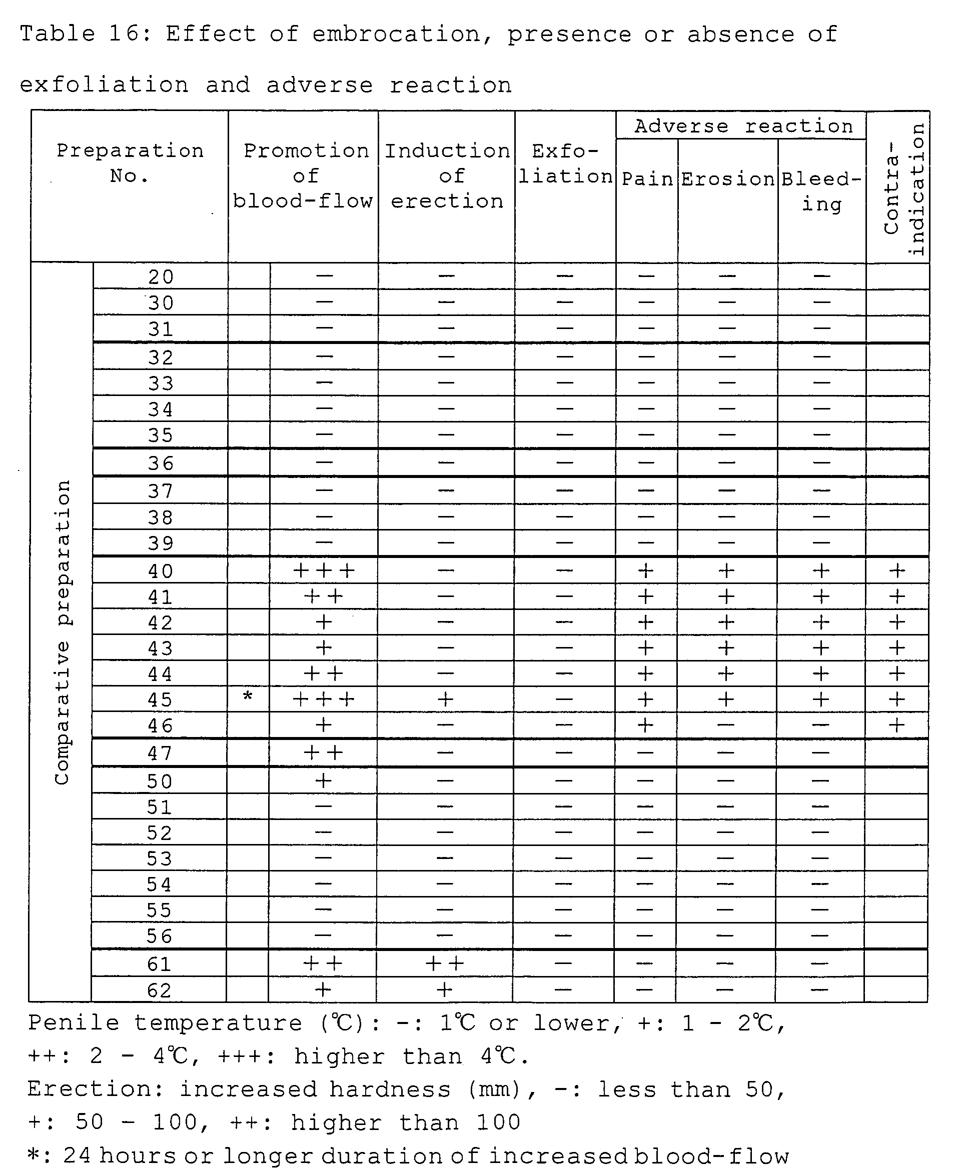
   From the results described above, the preparation of the present invention was regarded as a clinically useful agent effective by administration once a day for preventing or treating penile erectile dysfunction, or as an agent for improving a problem of coital dysfunction,
   wherein the preparation of the present invention has the effects of promoting penile blood-flow and increasing its hardness and duration of the effect for more than 24 hours without peeling off and showing any adverse reaction.
   Among them, in case of penile embrocation, significant effect was brought about by the preparations containing seaweed extract, its component of fucoidan (and its component of uronic acid), sodium hyaluronate, sodium alginate or carboxymethyl cellulose as a gelling agent, or by the above preparations added with hydrophilic petrolatum.
   In addition, sustained-release hydrogel preparations containing beraprost sodium and added with a cationic agent such as benzalkonium hydrochloride and tamsulosin hydrochloride or an anionic agent such as minodronic acid and sodium oleate are described in the publication of WO 99-33491, however, the preparations formulated with these agents (preparation No. 40 to 46) brought risks such as pain, erosion and bleeding, and therefore, such preparations were contraindication to a clinical application.
7) Studies on the adverse reaction in relation to intra-urethral infusion

By the same way as described in the previous section, feeling of incomplete urination, pain, bleeding and contraindication were studied with regard to administration by intra-urethral infusion. The results are shown in Table 17 together with the summary of blood-flow promotion and induction of penile erection.

In addition, as to the preparations added with an anionic detergent such as sodium benzalkonium, a study of this title was recessed because adverse reaction was observed in the previous embrocation studies.

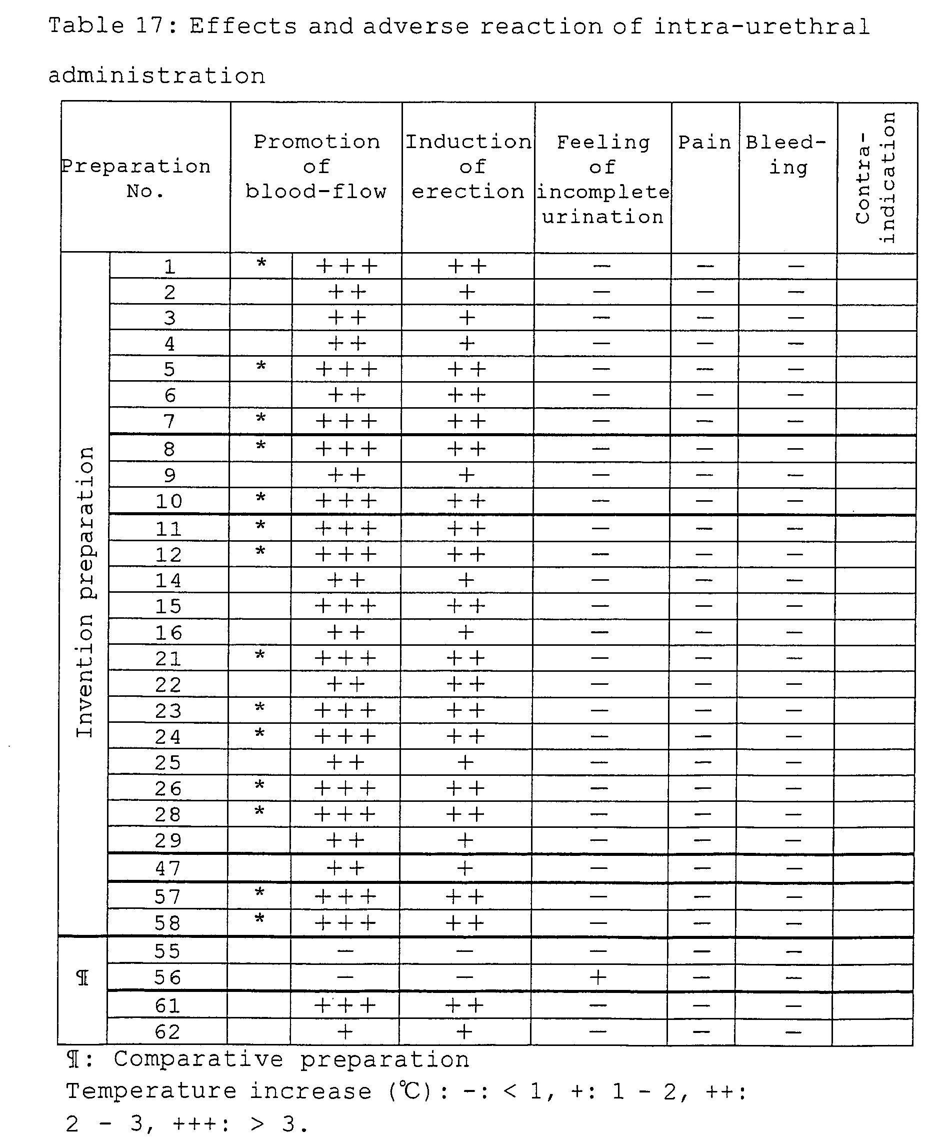

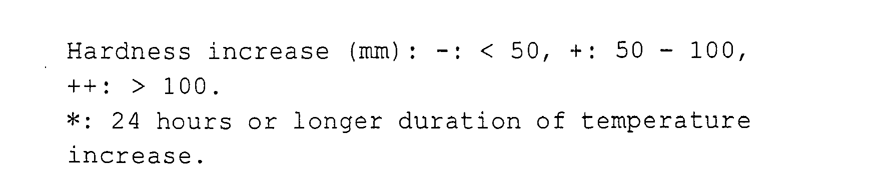

As the results described above, the preparation of the present invention acted to increase penile blood-flow and its hardness and duration of the effect for more than 24 hours without showing any adverse reaction. In this case as well, significant effect was brought about by the preparations containing seaweed extract, fucoidan, sodium hyaluronate and sodium alginate as a gelling agent, and more significant effect by the above preparations added with hydrophilic petrolatum.

### Example 4

### Preparation of a powdered gel preparation

Formulation of a powder preparation was performed with respect to the representative combination of the ingredients for the above described gel preparation which brought about effectiveness when administrated.
(1) Beraprost sodium, sodium alginate and lactose (a diluting agent) were mixed in weight ratio of 0.0002:0.5:9.5, and made into fine particles havig the size of 5-30 micons using a homogenizer (preparation No.63).
(2) By the same way, beraprost sodium, sodium hyaluronate and lactose were mixed in weight ratio of 0.0002:0.5:9.5, and made into fine particles using a homogenizer (preparation No. 64).
(3) One (1) ml of seaweed extract was dried in vacuo to make 0.2 g of a dry powder. Then, beraprost sodium, sodium alginate, powdered seaweed extract and lactose were mixed in weight ratio of 0.0002:0.5:0.5:9.0, and made into fine particles by the same procedure as described above (preparation No.65).
(4) Beraprost sodium and lactose were mixed in weight ratio of 0.002:10, and made fine particles by the same procedure as described above (preparation No.66).

### Example 5

### Administration of the powder preparation

(1) The powder preparation was embrocated on the surface of a penis using a cosmetic powder-puff. The embrocated powder is gelatinized gradually by the secretion from the neck region of a glans. Addition of a small amount of water, if needed, will bring about reduction of frictional resistance.
(2) A spray apparatus for the powder preparation was devised as shown in Fig. 11.
   In Fig. 11, 1 is a reservoir cell for the powder preparation, 2 is a knob of transferring disk-arrayed-cells one by one, and when a cell is shifted, a thin film sealing both side of the cell is cut and opened by a blade 3. By pushing a button 4, a biocompatible gas is belched from a gas container 5, and by that the powder preparation in a cell is sprayed out through a tubing 6 (may be plastic or metal).
   Cell volume is 0.1 to 1 ml, preferably 0.25 to 0.5 ml. Cell number is 1 to 100, preferably 10 to 30. Instead of gas, a manual air compressor may be used. Quantitative administration may be performed using equipment shown in Fig. 11. Also, intra-urethral administration may be possible. In this case, the powder preparation is also gradually gelatinized by the secretion.

### Example 6

### Clinical study on administration of the powder preparation

To the same cases that have received the gel preparations, 0.25 g of the powder preparation (preparation No. 63 to 66) was applied on a penis using a cosmetic puff. The results are shown in Table 22. As shown in Table 22, almost the same effects as observed with the gel preparations were obtained. No adverse reaction was observed.

**Table 22: The maximal increase of penile temperature, hardness and oxygen saturation after embrocation with the powder preparations**

| Preparation No. | Penile temperature (°C) | Duration of increased temperature (hr) | Hardness (mm) | Oxygen Saturation (%) |
|---|---|---|---|---|
| 6 3 | 4.4 | 24< | 119 | 46 |
| 6 4 | 4.6 | 24< | 120 | 43 |
| 6 5 | 4.8 | 24< | 130 | 45 |
| 6 6 | 4.9 | 12-24 | 110 | 4.7 |

### Industrial Applicability

The hydrogel preparation of the present invention is an external topical preparation to be administered to a penile region, which can be absorbed favorably through the skin or the mucous membrane of a glans and a penis and promotes blood-flow in a glans and a penis, induces penile erection and, at the same time, provides excellent moisturizing effect and reducing effect against frictional resistance.

Therefore, use of this drug may enables to prevent or treat male penile erectile dysfunction and, at the same time, overcome a problem of female insertion impediment caused by increased resistance due to vaginal secretion disorder. Namely, a sexual life of patients (including aged persons) having coital dysfunction can be normalized and thus QOL can be improved.

## Claims

1. An external topical hydrogel preparation comprising a prostaglandin I₂ derivative for prevention or treatment of penile erectile dysfunction.

2. An external topical hydrogel preparation comprising a prostaglandin I₂ derivative for improving coital dysfunction.

3. The preparation according to claim 1 or 2, wherein the prostaglandin I₂ derivative is a 4,8-inter-m-phenylenePGI₂ derivative.

4. The preparation according to claim 1 or 2, wherein the prostaglandin I₂ derivative is beraprost sodium.

5. The preparation according to any one of claims 1 to 4, wherein said preparation comprises one or more members of gelling agents selected from seaweed extracts, mucopolysaccharides, animal protein and a cellulose derivative.

6. The preparation according to any one of claims 1 to 4, wherein said preparation comprises one or more members of gelling agent selected from alginate, kelp extarct, fucoidan, uronic acid, chondroitin sulfate, hyaluronate, gelatin, atelocollagen and carboxymethylcellulose.

7. The preparation according to any one of claims 1 to 6, wherein said preparation further comprises hydrophilic petrolatum.

8. The preparation according to any one of claims 1 to 7, wherein said preparation further comprises inorganic salts.

9. The preparation according to any one of claims 1 to 8, wherein said preparation further comprises one or more members selected from a androgenic hormone preparation, an aphrodisiac drug, a spermatogenic promoter, a nitric monoxide-producing agent, amino acids and a phosphodiesterase 5 inhibitor.

10. The preparation according to any one of claims 1 to 9, wherein said preparation is administered to a penile area.

11. The preparation according to any one of claims 1 to 9, wherein said preparation is a liniment or an agent for intra-urethral infusion.

12. The preparation according to any one of claims 1 to 11, wherein said preparation is a powdered gel preparation.

13. Use of a hydrogel which ocmprises a prostaglandin I₂ derivative for the production of an external topical preparation for prevention or treatment of penile erectile dysfunction.

14. A method for treatment of penile erectile dysfunction, **characterized in that** the hydrogel which contains a prostaglandin I₂ derivative is administered to a penis or a urethra of a subject.
